# EUROPÄISCHE PATENTANMELDUNG

(11) **EP 0 129 182 A1**
(43) Veröffentlichungstag der Anmeldung: **27.12.1984**
(21) Anmeldenummer: 84106660.8
(22) Anmeldetag: 12.06.1984
(51) Int. Cl.: C08J 9/10, C07C 143/825

(54) **Arylsulfonylhydrazone als Treibmittel**

(30) Priorität: 21.06.1983 DE 3322320
(71) Anmelder: BAYER AG, 51368 Leverkusen (DE)
(72) Erfinder: Roos, Ernst, Dr., D-5068 Odenthal (DE); Kircher, Klaus, Dr., D-5090 Leverkusen 1 (DE)

(57) **Zusammenfassung**

Verwendung von Arylsulfonylhydrazonen der allgemeinen Formel 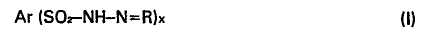 worin
Ar für einen gegebenenfalls durch Methyl und/oder Chlor substituierten Benzol-, Naphthalin-, Diphenyl-, Diphenylether-, Diphenylsulfon- oder Diphenylenoxidrest,
R für den Rest eines Aldehyds oder Ketons ohne das carbonyl-Sauerstoffatom, und
X für 1 oder 2
steht, als Treibmittel zur Herstellung von zelligen oder porösen Kunststoffartikeln.

## Beschreibung

Gegenstand der Erfindung ist die Verwendung von Arylsulfonylhydrazonen der allgemeinen Formel 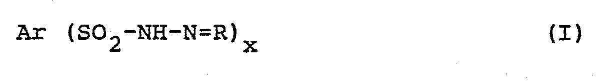 worin
Ar für einen gegebenenfalls durch Methyl und/oder Chlor substituierten Benzol-, Naphthalin-, Diphenyl-, Diphenylether, Diphenylsulfon- oder Diphenylenoxidrest,
R für den Rest eines Aldehyds oder Ketons ohne das Carbonyl-Sauerstoffatom und
x für 1 oder 2 steht,

als Treibmittel zur Herstellung von zelligen oder porösen Artikeln, insbesondere von Schaumstoffen aus thermoplastischen Kunststoffen.

Es ist bekannt, Schaumstoffe aus thermoplastischen Kunststoffen herzustellen, indem man in den Kunststoff ein organisches Treibmittel einarbeitet oder auf ein Kunststoffgranulat ein Treibmittel aufpudert, das sich bei Temperaturen, bei denen der Kunststoff plastisch erweicht, unter Gasentwicklung zersetzt.

Für diesen Zweck sind verschiedenartige Verbindungen vorgeschlagen worden (vgl. Angewandte Chemie 64 (1952) Seite 65 - 76).

Technisch werden zur Zeit vor allem das Azodicarbonamid und aromatische Sulfonhydrazide als Treibmittel verwendet; für einige Kunststoffe sind sie jedoch nicht geeignet. Vor allem Estergruppen enthaltende Kunststoffe werden durch die NH₂-Gruppen dieser Treibmittel leicht abgebaut, was zur Verschlechterung der mechanischen Eigenschaften dieser Kunststoffe führt.

Bei Verwendung von Azodicarbonamid enthalten die Zersetzungsgase auch Ammoniakgas, welches zu Korrosion der Metallformen und sonstiger Apparateteile sowie zu Geruchsbelästigungen führen kann. Bei Verwendung von aromatischen Sulfohydraziden als Treibmittel kann es zu Geruchsbelästigungen durch Schwefelverbindungen in den Zersetzungsprodukten kommen. Darüber hinaus liegt aber auch der Zersetzungspunkt der aromatischen Sulfohydrazide für Kunststoffe, die bei höheren Temperaturen verarbeitet werden müssen, z.B. Polycarbonate, Polyterephthalate und auch Kunststofflegierungen, die solche Polyester enthalten, für die Ausbildung eines gleichmäßigen Schaumstoffes zu niedrig.

Die erfindungsgemäßen Arylsulfonylhydrazone der Formel I sind von diesen Nachteilen frei.

Sie können hergestellt werden durch Umsetzung von Arylsulfohydraziden (A) mit Aldehyden oder Ketonen (B) unter Wasserabspaltung gemäß: 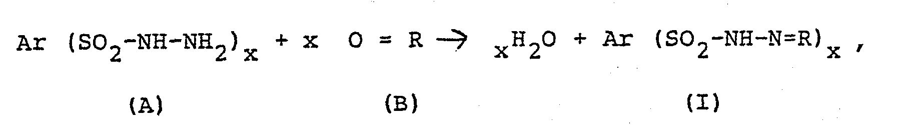 wobei Ar, R und x die oben angegebene Bedeutung haben.

Insbesondere ist R ein Alkylrest mit 1 - 8 C-Atomen oder ein Arylrest mit 6 - 12 C-Atomen.

Beispielsweise können die Komponenten A und B mit oder ohne Lösungsmittel umgesetzt werden, wobei das entstehende Wasser durch Destillation unter normalem oder vermindertem Druck und auch durch azeotrope Destillation entfernt werden kann.

Die Reaktionstemperaturen sind im allgemeinen 0 bis 100°C, bevorzugt 15 bis 80°C. Die Reaktanten werden im allgemeinen in etwa stöchiometrischen Mengen eingesetzt. Ein Überschuß eines Reaktanten bis etwa zur doppelten stöchiometrischen Menge ist möglich.

Wenn die Umsetzung in einem Lösungsmittel durchgeführt wird, sind insbesondere polare Lösungsmittel verwendbar. Die Menge der Lösungsmittel wird so bemessen, daß gut rührbare Suspensionen der Ausgangsprodukte entstehen.

Beispiele für geeignete Ausgangsprodukte als Arylsulfohydrazide sind:
Benzolsulfohydrazid, o-, m- und p-Toluolsulfohydrazid, p-tert.₋Butyl-benzolsulfohydrazid, o-, m-, p-Chlorbenzolsulfohydrazid, 5-Chlor-2-methyl-benzolsulfohydrazid, 3-Chlor-4-methyl-benzolsulfohydrazid, 2-Chlor-4-methyl- benzolsulfohydrazid, Naphthalin-1-sulfohydrazid, Naphthalin-2-sulfohydrazid, Diphenyl-4-sulfohydrazid, Benzol-1,3- disulfohydrazid, Naphthalin-1,4-disulfohydrazid, Naphthalin-1,5-disulfohydrazid, Naphthalin-2,6-disulfohydrazid, Diphenyl-1,4-disulfohydrazid, Diphenylether₋4,4'-disulfohydrazid, Ditoluylether-disulfohydrazid, Diphenylsulfon-3,3'- disulfohydrazid, Diphenylenoxid-4,4'-disulfohydrazid.
Beispiele für geeignete Aldehyde und Ketone sind solche mit aliphatischen, cycloaliphatischen und/oder aromatischen Resten, wobei die Gesamtzahl der Kohlenstoffatome 10 nicht überschreiten sollte. Im einzelnen werden genannt:
Acetaldehyd, Propionaldehyd, n-Butyraldehyd, Isobutyraldehyd, Heptanal, 2-Ethylhexanal, Tetrahydrobenzaldehyd, Hexahydrobenzaldehyd, Benzaldehyd, o-, m-, p-Toluylaldehyd, Aceton, Butanon, 2-Pentanon, 3-Pentanon, 3-Methyl-2-butanon, 4-Methyl-2-pentanon, 4-Heptanon, 5-Methyl-3-heptanon, 2,6-Dimethyl-4-heptanon, Acetophenon, Propiophenon, Tetralon, Cyclohexanon, o-, m-, p-Methyl-cvclohexanon, 3,3,5-Trimethyl-cyclohexanon.

Bevorzugt ist die Umsetzung der Komponenten A und B in einem niedrigsiedenden, mit Wasser mischbaren Alkohol, wie z.B. Methanol oder Ethanol,gegebenenfalls bei 15°C bis 80°C Temperatur.

Als Beispiele für die erfindungsgemäBen Treibmittel werden im folgenden einige Arylsulfonylhydrazone aufgeführt:

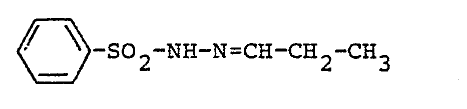

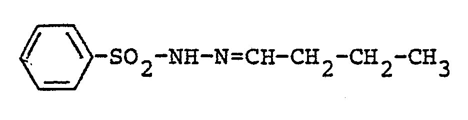

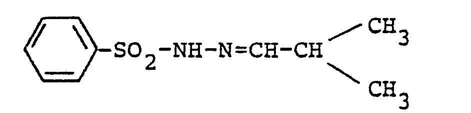

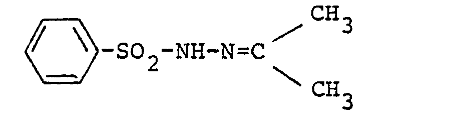

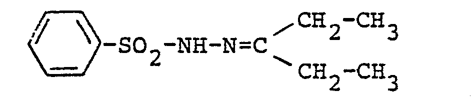

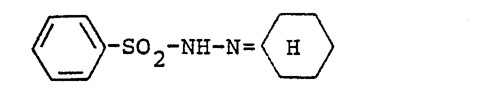

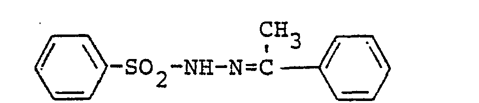

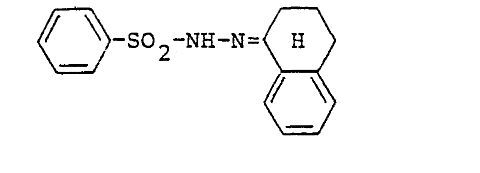

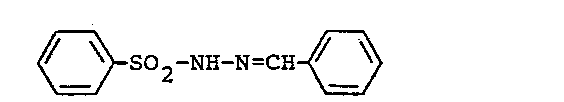

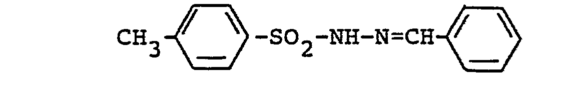

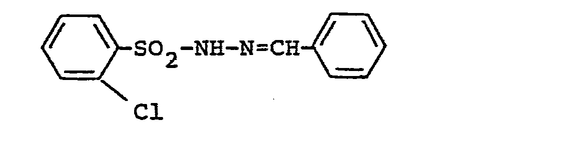

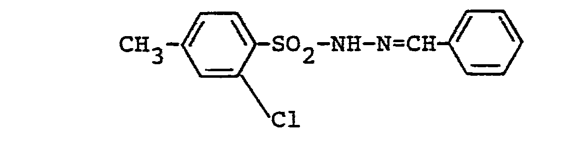

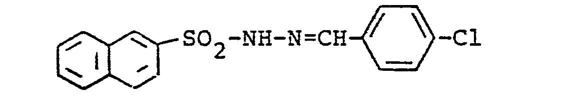

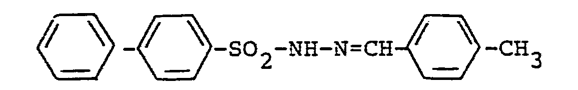

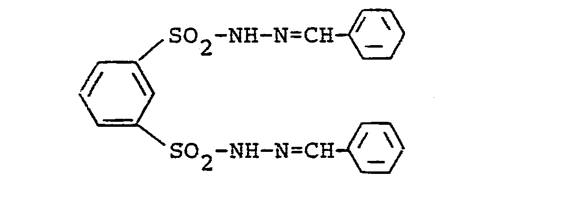

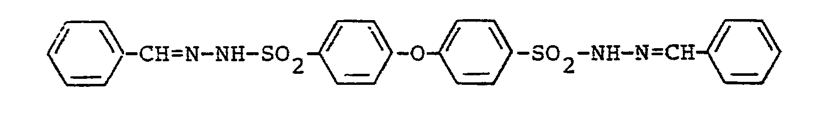

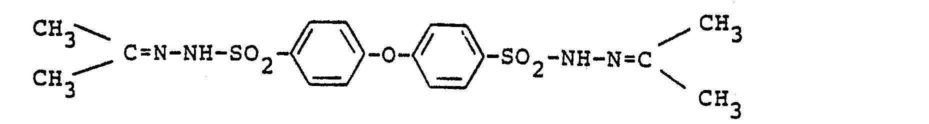

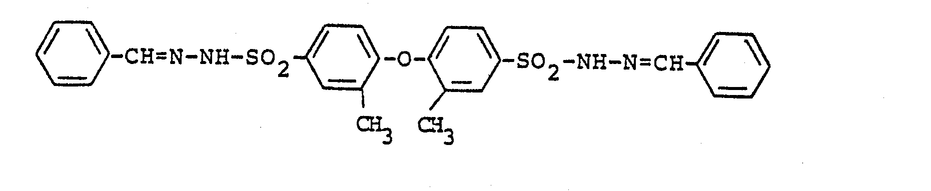

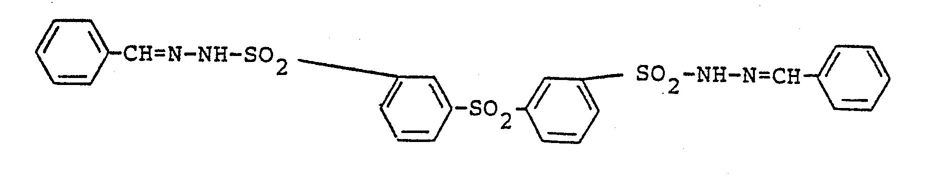

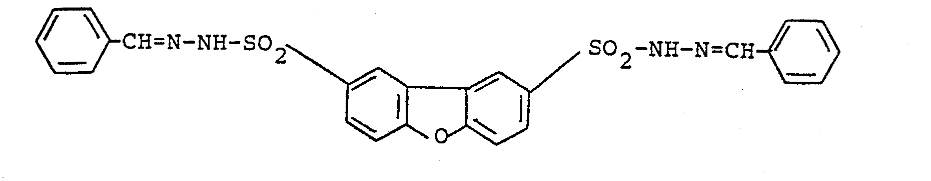

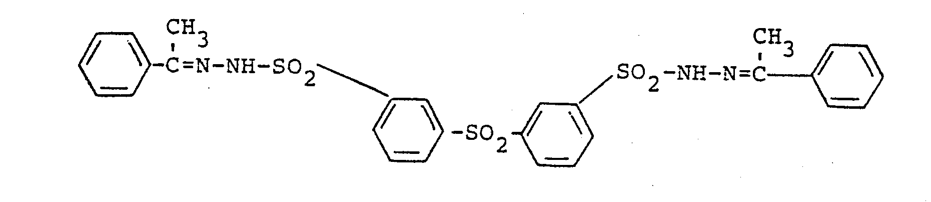

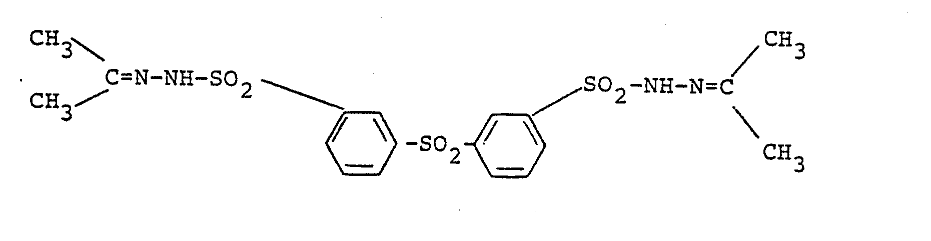

Die Arylsulfonylhydrazone der Erfindung sind als Treibmittel für thermoplastische Kunststoffe, z.B. Vinylpolymerisate und Polykondensate geeignet. Bevorzugt wird jedoch ihr Einsatz in Kunststoffen, welche Estergruppen enthalten, z.B. in Polycarbonaten und polyestern sowie in Kunststofflegierungen, welche Polycarbonate und Polyester enthalten. Als Polycarbonate werden insbesondere solche auf Basis von Bisphenol A und als Polyester solche auf Basis von Polybutylenterephthalat bevorzugt.

Die erfindungsgemäß zu verwendenden Treibmittel können in die zu verschäumenden Mischungen auf übliche Weise in einer Menge von etwa 0,2 bis 1 Gew.-% eingemischt werden, beispielsweise auf Mischwalzwerken, Innenmischern oder in mit Rührwerken ausgestatteten Mischbehältern; sie können gleichzeitig mit der Zugabe anderer Mischungsbestandteile zugesetzt werden.

Die Mischungen können auch andere bekannte Hilfsstoffe enthalten, z.B. Füllstoffe, Antioxic=ntien, Stabilisatoren, Wachse, Farbstoffe, Pigmente, Weichmacher, Gleitmittel und Peroxide.

### Beispiele für die Herstellung von Aryl-monosulfonylhydrazonen und Aryl-bis-sulfonylhydrazonen

### Beispiel 1

N-Benzyliden-N'-benzolsulfonyl-hydrazin:

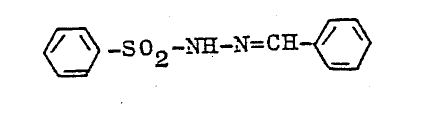

₁₇₂ g (1 Mol) Benzolsulfohydrazid in 500 ml Methanol wurden bei 20 bis 40°C mit 106 g (1 Mol) Benzaldehyd versetzt. Das Gemisch wurde 4 Stunden unter Rückfluß gekocht und nach dem Erkalten wurden die ausgeschiedenen Kristalle abgesaugt, mit Methanol und Wasser gewaschen und in einem Umlufttrockenschrank bei 60 bis 70°C getrocknet.

Ausbeute: 222 g = 85,3 % der Theorie an farblosen Kristallen vom Fp 110-112°C.

Aus der Mutterlauge kann eine weitere Kristallfraktion vom Fp. 108-110°C erhalten werden.

### Beispiel 2

N,N'-Bis-benzyliden-benzol-1,3-disulfonylhydrazin:

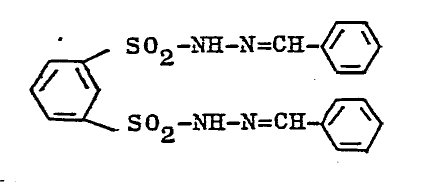

47 g Benzol-1,3-disulfohydrazid, 300 ml Methanol und 37,5 g Benzaldehyd wurden 4 Stunden unter Rückfluß gekocht. Nach dem Erkalten wurden die ausgeschiedenen Kristalle abgesaugt und getrocknet.

Es wurden 70 g farblose Kristalle vom Fp. 182°C erhalten.

### Beispiel 3

N,N'-Bis-benzyliden-diphenyloxid-1,4-disulfonylhydra- zin:

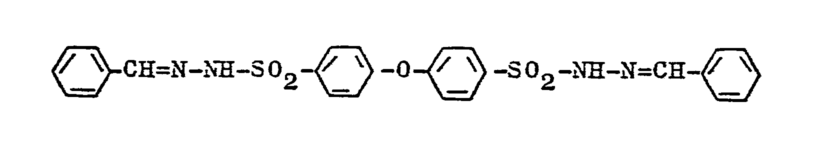

143 g Diphenyloxid-4,4'-disulfohydrazid, 600 ml Methanol und 85 g Benzaldehyd wurden 4 Stunden unter Rückfluß gekocht. Nach Abdestillieren von etwa 300 ml Methanol-Wasser-Gemisch und Abkühlen wurden die ausgeschiedenen Kristalle abgesaugt und getrocknet.

Ausbeute: 165 g fast farblose Kristalle, Fp. 110-111°C.

### Beispiel 4

### _{N},N'-Bis-benzyliden-diphenylenoxid-4,4'-disulfonylhydrazin

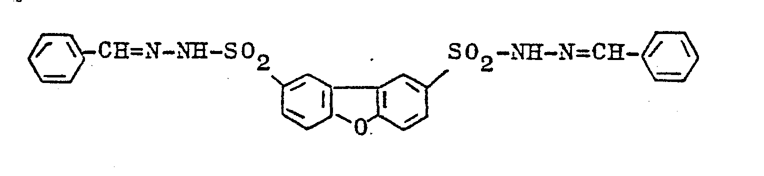

178 g Diphenylenoxid₋4,4'-disulfohydrazid, 800 ml Methanol und 106 g Benzaldehyd wurden 4 Stunden unter Rückfluß gekocht. Nach Abkühlen auf 0°C wurde der Kristallbrei abgesaugt und getrocknet.

Ausbeute: 218 g fast farblose Kristalle, Fp. 207°C.

### Beispiel 5

### N,N'-Bis-benzyliden-2,2'-dimethyl-diphenyloxid-4,4'- disulfonylhydrazin

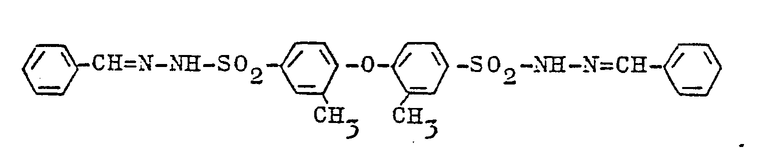

193 g 2,2'-Dimethyl-diphenyloxid-4,4'-disulfohydrazid, 800 ml Methanol und 106 g Benzaldehyd wurden 5 Stunden bei Raumtemperatur gerührt, wobei zunächst eine Lösung, dann ein Kristallbrei entstand. Dieser wurde bei 0°C abgesaugt und getrocknet.
Ausbeute: 265 g fast farblose Kristalle, Fp. 172°C.

### Beispiel 6:

### N,N'-Bis-benzyliden-diphenylsulfon-3,3'-disulfonyl- hydrazin

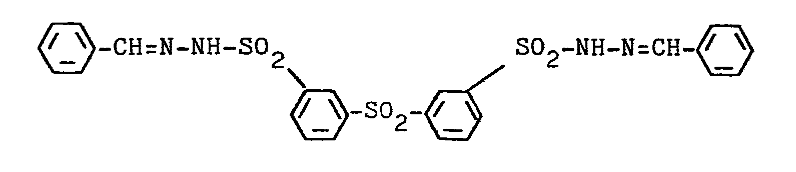

203 g Diphenylsulfon-3,3'-disulfohydrazid, 800 ml Methanol und 106 g Benzaldehyd wurden 4 Stunden unter Rückfluß gekocht. Nach dem Erkalten wurde der entstandene Kristallbrei abgesaugt und getrocknet.
Ausbeute: 250 g farbloses Kristallpulver, Fp. 204°C. ;

### Beispiel 7 (Vergleichsversuch)

ₐ) Ein Bisphenol-A-Polycarbonat mit einer Schmelzviskosität von 10 g / 10 min (300°C, 1,2 kg) wurde in einem Taumelmischer mit 0,35 Gew.-% handelsüblichem Azodicarbonamid (H₂N-CO-N=N-CO-NH₂) vermischt und anschließend zu Formkörpern verspritzt. Die relative Lösungsviskosität der Formkörper betrug 1,193.
b) Als Vergleich wurde das gleiche Polycarbonat, jedoch ohne den Zusatz an Azodicarbonamid unter gleichen Bedingungen zu Formkörpern verspritzt, wobei eine relative Lösungsviskosität von 1,286 resultierte.

Der durch das Treibmittel verursachte Molekulargewichtsabbau, charakterisiert durch den Abfall der relativen Lösungsviskosität von 1,286 (b) auf 1,193 (a), ist von einer Verminderung der Zähigkeitseigenschaften begleitet.

Die Schlagzähigkeit nach DIN 53 453 der Formkörper gemäß (a) ist kleiner als 100 kJ/m², wogegen der Formkörper gemäß (b) im Schlagversuch gemäß DIN 53 453 nicht bricht.

Der Rückgang der relativen Lösungsviskosität und der Zähigkeit zeigen, daß Treibmittel mit freien - NH₂-Gruppen hydrolysierbare Kunststoffe abbauen.

### Beispiel 8 (Vergleichsversuch)

Auf das Polycarbonat des Beispiels 7 wurde Oxalsäurebishydrazid in der in Tabelle 1 angegebenen Konzentration aufgetrudelt; die Mischung wurde durch Extrusion bei 240°C kompoundiert. Die erhaltenen Granulatkörner wurden bei 300°C zu Normkleinstäben der Dimension 60 mm x 6 mm x 4 mm verspritzt. Am Granulat sowie an den Prüfkörpern wurden die folgenden relativen Viskositäten gemessen:

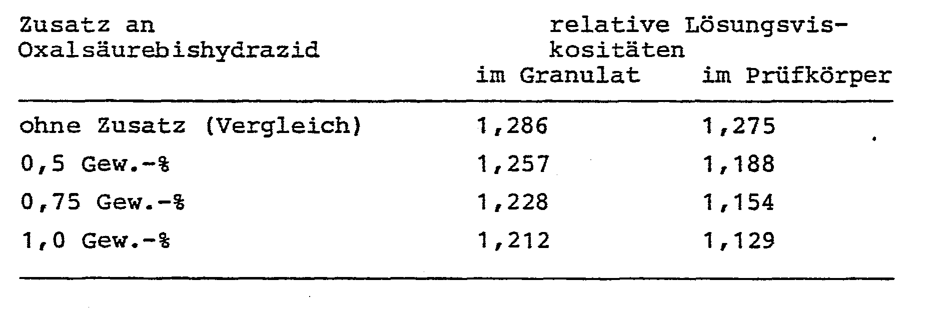

Oxalsäurebishycrazid führt offensichtlich zu einem ausgeprägten Molekulargewichtsabbau und zur Verschlechterung der mechanischen Eigenschaften.

In den folgenden erfindugnsgemäßen Beispielen wird gezeigt, daß Treibmittel auf der Basis von Hydrazon, wo keine basische Amingruppe vorliegt, keinen nennenswerten Einfluß auf die Molekulargewichte haben.

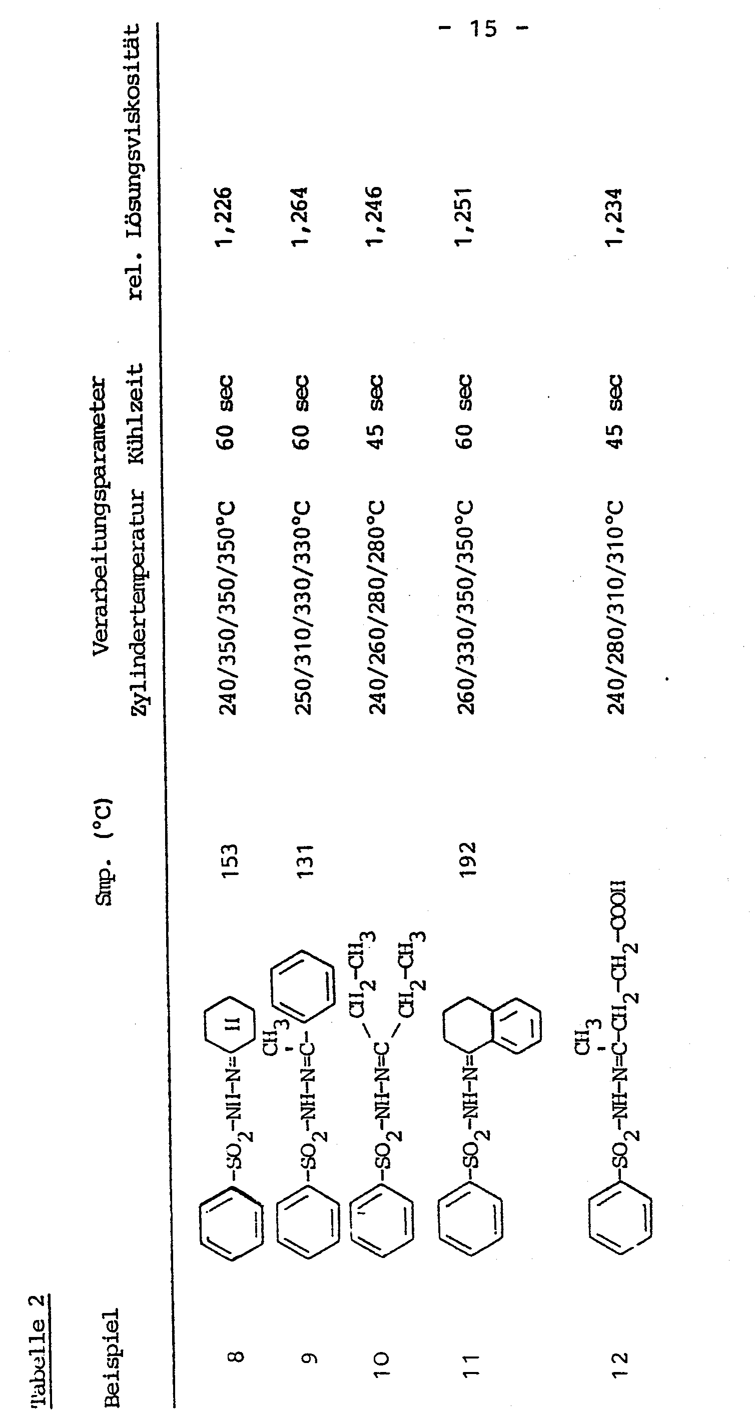

In den folgenden erfindungsgemäßen Beispielen wird gezeigt, daß Treibmittel auf der Basis von Hydrazonen, wo keine basische Amingruppe vorliegt, keinen nennenswerten Einfluß auf die Molekulargewichte haben.

Die in Tabelle 2 angeführten Monosulfohydrazone wurden in einer Menge von 0,35 Gew.-% einzeln in einem Taumelmischer auf das Polycarbonat des Beispiels 7 aufgetrudelt, so daß sich das Treibmittel auf der Oberfläche der einzelnen Granulat-Körner gleichmäßig verteilte.

Das mit Treibmittel überzogene Granulat wurde auf einer Mannesmann-Demag DNC II 80-Spritzgußmaschine zu Rechteckplatten der Abmessung 150 mm x 90 mm x 8 mm und einem Raumgewicht von 0,9 g/cm³ verspritzt.

Maschinenparameter: Verschlußdüse, Schneckendrehzahl 18 U/min; Spritzdruck 127 bar (maximal); Nachdruck: 30 bar; Staudruck: 15 bar; Pausenzeit: 3 sec; Werkzeugtemperatur: 50°C. Die für eine optimale Schaumherstellung erforderlichen Zylindertemperaturen sind Treibmittelabhängig und in Tab. 1 aufgelistet. Alle Produkte konnten zu einem Polycarbonat-Formteil verarbeitet werden; da ohne Nucleierungshilfsmittel gearbeitet wurde, war der Schaum grobporig.

Der Molekulargewichtsabbau, charakterisiert durch die Abnahme der relativen Lösungsviskosität von 1,290 im Ausgangsgranulat wird nur wenig beeinflußt, wobei insbesondere die aromatischen Sulfonylhydrazone einen recht geringen Einfluß auf den MG-Abbau haben.

### Beispiel 13

Ein Polycarbonat mit einer Schmelzviskosität wie im Beispiel 7 und einem Gehalt von 5 Gew.-% Glasfaser (Raumgewicht 1,240) wurde mit 0,3 Gew.-% der Verbindung

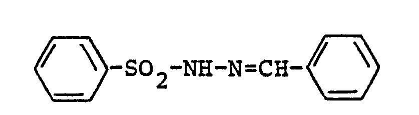

Smp. 112°C, Gasausbeute bei 260°C, 90 ml/g durch Auftrudeln in einem Taumelmischer versetzt.

Die Mischung wurde wie in Beispiel 8 bis 12, jedoch mit 240/300/320/320/320 (Düse) °C als Zylindertemperaturen zu feinzelligen und hellen Rundscheiben der Dimension 200 mm x 8 mm und der Dichte 0,9 g/cm verschäumt.

Die relative Lösungsviskosität betrug am Spritzling 1,282 und am Granulat 1,295, was eine Abnahme von nur 0,013 Einheiten entspricht. Die Schlagzähigkeit des Schaumteils nach DIN 53 453 betrug 37,1 kJ/m².

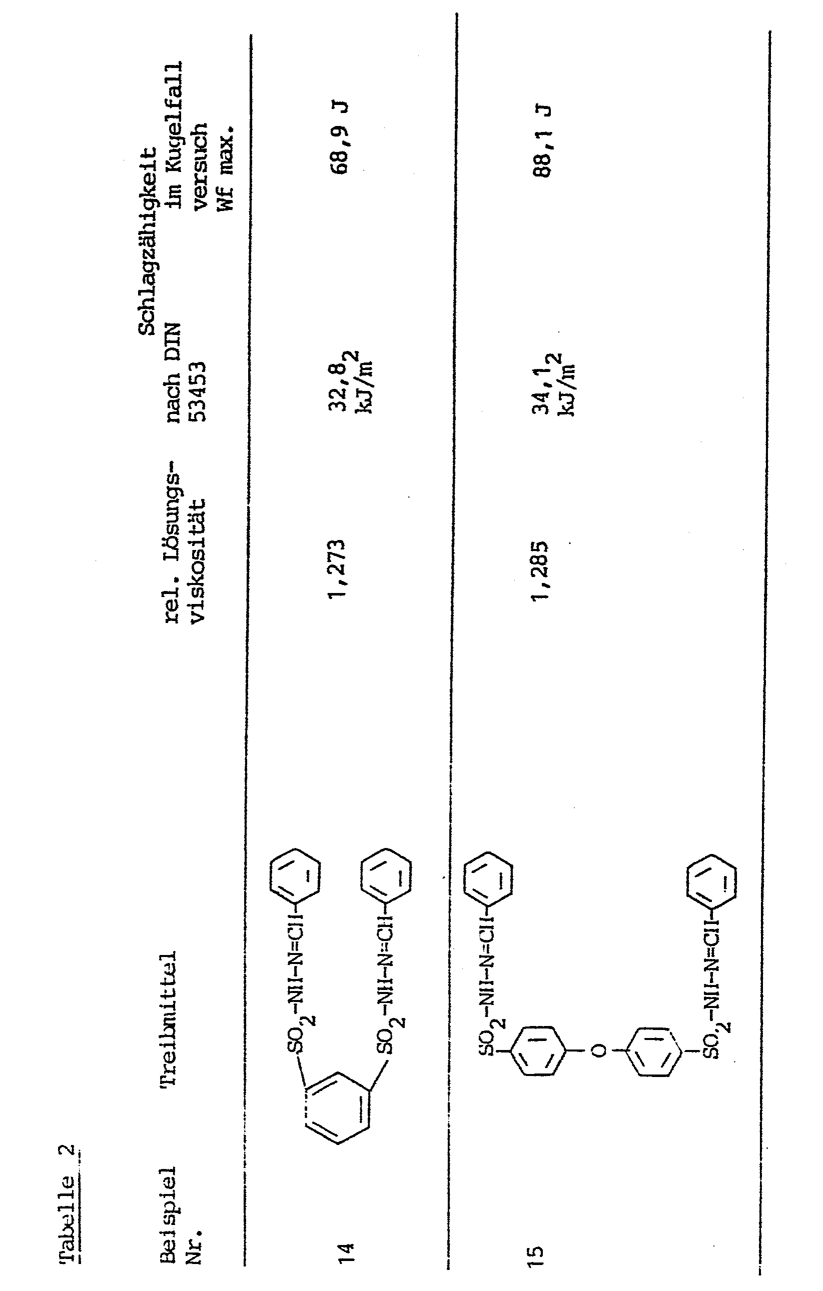

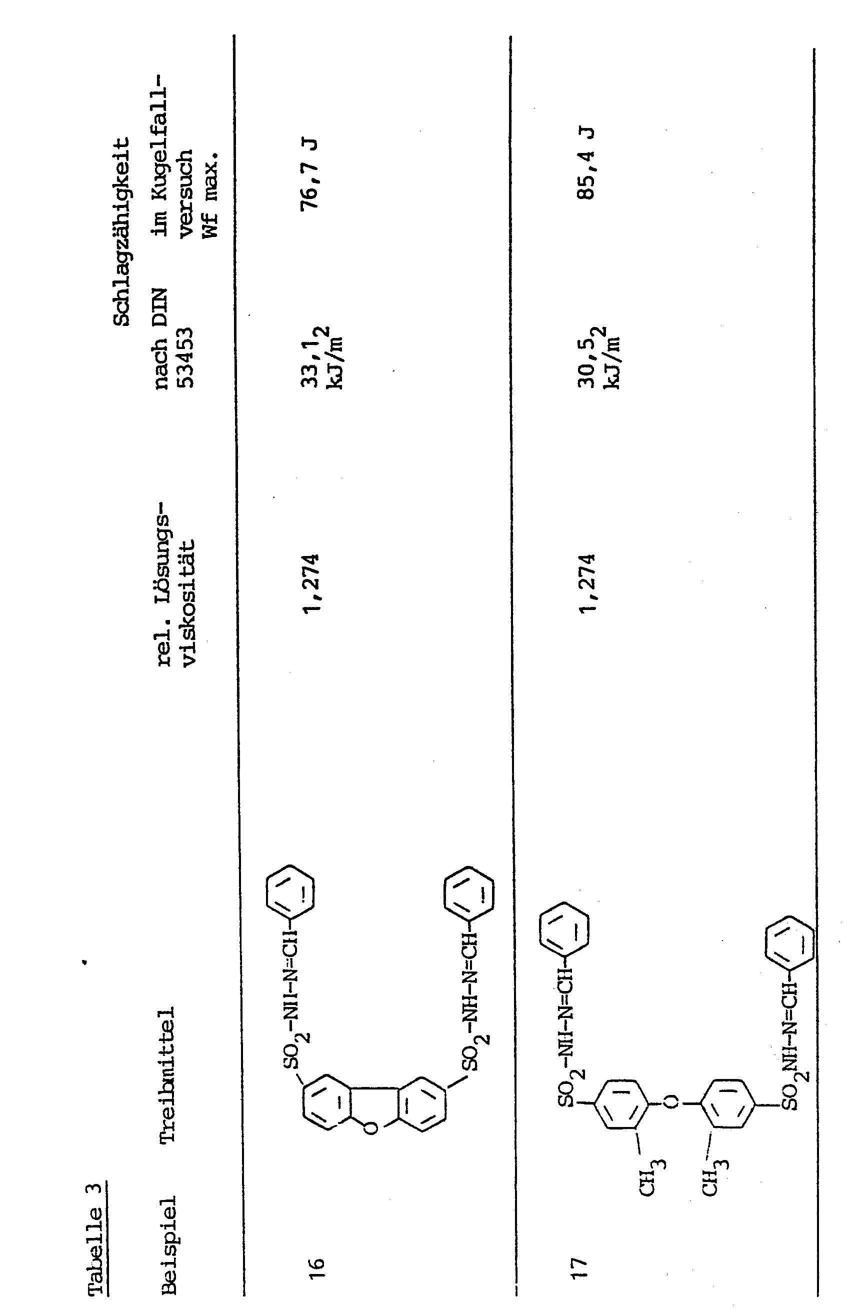

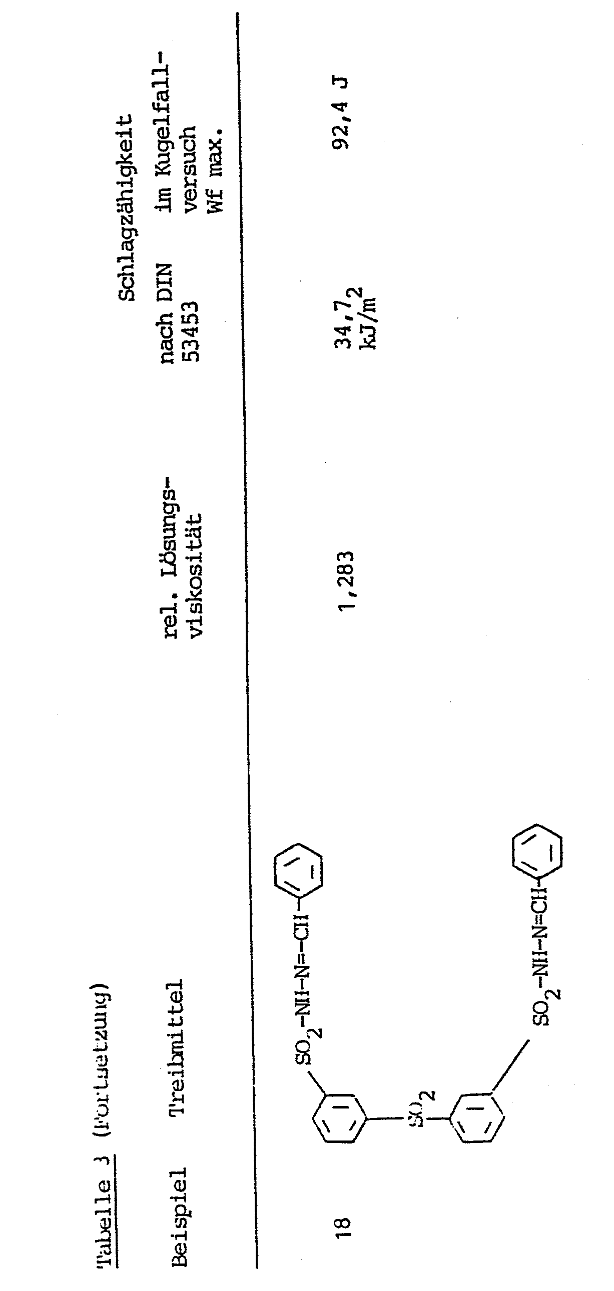

Die in Tabelle 3 angeführten Bis-sulfonylhydrazone wurden in einem Taumelmischer auf das Polycarbonat mit 5 % Glasfasern des Beispiels 15 gegeben. Die Kunststoff-Treibmittel-Gemische wurden bei Zylindertemperaturen von 240/280/330/330°C (Düse) zu Formteilen,wie in Beispiel 9 beschrieben,verarbeitet. Es resultierten in allen Fällen helle, feinzellige Integralschaum-Formteile mit den in Tabelle 3 angegebenen Eigenschaften.

## Patentansprüche

1. Verwendung von Arylsulfonylhydrazonen der allgemeinen Formel I 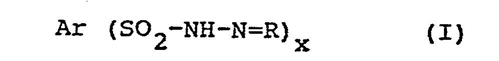 worin
Ar für einen gegebenenfalls durch Methyl und/oder Chlor substituierten Benzol-, Naphthalin-, Diphenyl-, Diphenylether-, Diphenylsulfon- oder Diphenylenoxidrest,
R für den Rest eines Aldehyds oder Ketons ohne das Carbonyl-Sauerstoffatom, und
x für 1 oder 2
steht, als Treibmittel zur Herstellung von zelligen oder porösen Kunststoffartikeln.

2. Verwendung von N,N'-Bis-benzyliden-diphenylsulfon-3,3'-disulfonylhydrazin gemäß Anspruch 1.

3. Verwendung der Arylsulfonylhydrazone gemäß Anspruch 1 in Polycarbonat als Kunststoff.

4. Verwendung der Arylsulfonylhydrazone gemäß An- spruch 1 in Polybutylenterephthalat als Kunststoff.

5. N,N'-Bis-benzyliden-diphenylsulfon-3,3'-disulfonyl- bydrazin.
